# EUROPÄISCHE PATENTSCHRIFT

(11) **EP 2 320 804 B1**
(45) Veröffentlichungstag und Bekanntmachung des Hinweises auf die Patenterteilung: **06.04.2016**
(21) Anmeldenummer: 09745657.8
(22) Anmeldetag: 29.04.2009
(51) Int. Cl.: A61B 90/98, A61B 17/00

(54) **CHIRURGISCHE ANTRIEBSEINHEIT, CHIRURGISCHES INSTRUMENT UND CHIRURGISCHES ANTRIEBSSYSTEM**
SURGICAL DRIVE UNIT, SURGICAL INSTRUMENT, AND SURGICAL DRIVE SYSTEM
UNITÉ D'ENTRAÎNEMENT CHIRURGICALE, INSTRUMENT CHIRURGICAL ET SYSTÈME D'ENTRAÎNEMENT CHIRURGICAL

(30) Priorität: 14.05.2008 DE 102008024438
(43) Veröffentlichungstag der Anmeldung: 18.05.2011
(73) Patentinhaber: Aesculap AG, 78532 Tuttlingen (DE)
(72) Erfinder: HAFNER, Ronald, 88637 Leibertingen (Thalheim) (DE); MOOSMANN, Ernst, 88637 Leibertingen-Altheim (DE); SCHNEIDER, Jürgen, 78532 Tuttlingen (DE); SCHNELL, Birgit, 78579 Neuhausen (DE)
(74) Vertreter: Hoeger, Stellrecht & Partner Patentanwälte mbB
(86) Internationale Anmeldenummer: PCT/EP2009/055239
(87) Internationale Veröffentlichungsnummer: WO 2009/138324

(56) Entgegenhaltungen:
- EP-A- 0 788 778
- EP-A- 2 042 120
- WO-A-98/06338
- DE-A1- 10 225 857
- US-A1- 2004 209 223
- US-A1- 2004 220 602

## Beschreibung

Die vorliegende Erfindung betrifft eine chirurgische Antriebseinheit eines chirurgischen Instruments, welche einen Motor mit mindestens zwei Motorwicklungen und eine Speichereinrichtung zur Speicherung von die Antriebseinheit und/oder den Motor charakterisierenden Daten umfasst, wobei die Antriebseinheit eine erste Sende- und Empfangseinrichtung, welche mit der Speichereinrichtung verbunden ist, und eine zweite Sende- und Empfangseinrichtung, welche mit mindestens zwei Anschlusskontakten des Motors verbunden ist, umfasst und dass die erste und die zweite Sende- und Empfangseinrichtung derart ausgebildet sind und zusammenwirken, dass Daten berührungslos zwischen ihnen übertragbar sind.

Ferner betrifft die vorliegende Erfindung ein chirurgisches Instrument umfassend eine Antriebseinheit und ein mit der Antriebseinheit verbundenes oder verbindbares und von der Antriebseinheit antreibbares chirurgisches Werkzeug, wobei die Antriebseinheit einen Motor mit mindestens zwei Motorwicklungen und eine Speichereinrichtung zur Speicherung von die Antriebseinheit und/oder den Motor charakterisierenden Daten umfasst, wobei die Antriebseinheit eine erste Sende- und Empfangseinrichtung, welche mit der Speichereinrichtung verbunden ist, und eine zweite Sende- und Empfangseinrichtung, welche mit mindestens zwei Anschlusskontakten des Motors verbunden ist, umfasst und dass die erste und die zweite Sende- und Empfangseinrichtung derart ausgebildet sind und zusammenwirken, dass Daten berührungslos zwischen ihnen übertragbar sind.

Des Weiteren betrifft die vorliegende Erfindung ein chirurgisches Antriebssystem umfassend mindestens eine Steuer- und/oder Regelungseinrichtung und mindestens zwei mit dieser verbindbare und ansteuerbare chirurgische Antriebseinheiten oder chirurgische Antriebseinheiten umfassende chirurgische Instrumente, wobei mindestens eine der mindestens zwei chirurgischen Antriebseinheiten einen Motor mit mindestens zwei Motorwicklungen und eine Speichereinrichtung zur Speicherung von die Antriebseinheit und/oder den Motor charakterisierenden Daten umfasst, wobei die Antriebseinheit eine erste Sende- und Empfangseinrichtung, welche mit der Speichereinrichtung verbunden ist, und eine zweite Sende- und Empfangseinrichtung, welche mit mindestens zwei Anschlusskontakten des Motors verbunden ist, umfasst und dass die erste und die zweite Sende- und Empfangseinrichtung derart ausgebildet sind und zusammenwirken, dass Daten berührungslos zwischen ihnen übertragbar sind.

Vorrichtungen der eingangs beschriebenen Art sind beispielsweise aus der DE 102 25 857 A1 bekannt. Die Antriebseinheiten beziehungsweise Motoren des dort beschriebenen Antriebssystems sind mit jeweils einer Speichereinrichtung ausgestattet, in der den Motortyp charakterisierende Daten abgespeichert sind. Diese Daten werden bei der Herstellung in der Speichereinrichtung hinterlegt und sind vom Verwender nicht änderbar. Bei Motoren mit drei Motorwicklungen werden zur Ansteuerung des Motors drei Anschlussleitungen benötigt, um den Motor mit einem Motorsteuergerät zu verbinden. Um den jeweiligen Motor charakterisierende Daten in das Steuergerät zu übertragen, sind weitere Steuer- und/oder Datenleitungen erforderlich. Folge hiervon ist, dass je nach Anforderung eine entsprechende Anzahl von Kontakten für die Anschlussleitungen des Motors und die Steuer- und/oder Datenleitungen vorzusehen ist. Da die Antriebseinheiten jedoch nach einem chirurgischen Eingriff gereinigt und gegebenenfalls dampfsterilisiert werden müssen, können Kontaktprobleme nicht ausgeschlossen werden, insbesondere bei den Steuer- und Datenleitungen, die nicht mit den Motorwicklungen verbunden werden.

Weitere Vorrichtungen der eingangs beschriebenen Art sind ferner aus der US 2004/209223 A1, der US 2004/220602 A1 sowie der WO 98/06338 A2 bekannt.

Es ist daher Aufgabe der vorliegenden Erfindung, die Zuverlässigkeit von Antriebseinheiten, Instrumenten und Antriebssystemen der eingangs beschriebenen Art zu verbessern.

Diese Aufgabe wird bei einer chirurgischen Antriebseinheit, bei einem chirurgischen Instrument und bei einem chirurgischen Antriebssystem der jeweils eingangs beschriebenen Art erfindungsgemäß dadurch gelöst, dass eine (72c) der mindestens zwei Motorwicklungen (72a, 72b, 72c) die zweite Sende- und Empfangseinrichtung (82b) bildet.

Die erfindungsgemäße Weiterbildung bekannter chirurgischer Antriebseinheiten, die insbesondere Teile eines chirurgischen Instruments sowie eines chirurgischen Antriebssystems sein können, hat insbesondere den Vorteil, dass Daten aus der Speichereinrichtung über die Anschlusskontakte, welche mit den mindestens zwei Motorwicklungen und gegebenenfalls mit Anschlussleitungen des Motors leitend verbunden sind, ausgelesen werden können. Zusätzliche Steuer- oder Datenleitungen sind zum Auslesen der Speichereinrichtung nicht erforderlich. Insbesondere bei neuen Motorgenerationen, die in Verbindung mit geeigneten Steuer- und/oder Regelungseinrichtungen eine Drehzahldetektion ohne Sensoren am Motor oder der Antriebseinheit ermöglichen und bei denen daher keine Daten- oder Steuerleitungen vorgesehen werden müssen, eignet sich die erfindungsgemäß vorgeschlagene Weiterbildung hervorragend, um gänzlich auf die Daten- oder Steuerleitungen zu verzichten. Die Abfrage des Motortyps kann dann beispielsweise erfolgen, indem vor Inbetriebnahme des Motors über die Motoranschlusskontakte- oder -leitungen, die mit der ersten Sende- und Empfangseinrichtung verbunden sind, berührungslos durch Datenaustausch mit der zweiten Sende- und Empfangseinrichtung Motortypdaten aus der Speichereinrichtung ausgelesen und beispielsweise an ein Motorsteuergerät übertragen werden können, um in diesem bestimmte Betriebsparameter in gewünschter Weise automatisch einzustellen. So kann vermieden werden, dass der Motor beispielsweise mit zu hohen Strömen bestromt und außerhalb eines zulässigen Drehzahlbereichs betrieben werden kann. In jedem Fall wird durch den erfindungsgemäß möglichen Verzieht auf Steuer- oder Datenleitungen die Zahl von Kontakten an der Antriebseinheit reduziert, welche infolge von Korrosion bedingt durch Reinigung und Sterilisierung eine dauerhafte Funktionssicherheit der Antriebseinheit infrage stellen können. Ein besonders einfacher Aufbau der Antriebseinheit lässt sich dadurch erreichen, dass eine der mindestens zwei Motorwicklungen die zweite Sende- und Empfangseinrichtung bildet. Der ausgewählten Motorwicklung kommt somit eine Doppelfunktion zu. Zum Einen ist sie Bestandteil des Motors als eigentlichem Antriebselement und zum Anderen bildet sie einen Teil einer Übertragungseinrichtung, um Daten aus der Speichereinrichtung beispielsweise zu einer Steuer- und/oder Regelungseinrichtung in Form eines Motorsteuergeräts zu übertragen. Dieser Aufbau gestattet es, die Anschlusskontakte beziehungsweise -leitungen zum Motor auch als Steuer- oder Datenleitungen zu nutzen, insbesondere dann, wenn der Motor nicht betrieben wird. Eine Verbindung der Steuer- und/oder Regelungseinrichtung mit der zweiten Sende- und Empfangseinrichtung kann somit direkt über die Motoranschlusskontakte, also über die Anschlusskontakte der ausgewählten Motorwicklung erfolgen.

Ein besonders einfacher und sicherer Datenaustausch ist möglich, wenn die erste und/oder die zweite Sende- und Empfangseinrichtung in Form einer Antenne ausgebildet sind.

Auf einfache Weise lassen sich beispielsweise Daten zur Charakterisierung des Motors, beispielsweise des Motortyps, von der Antriebseinheit an eine Steuer- und/oder Regelungseinrichtung übermitteln, wenn zwischen der ersten und zweiten Sende- und Empfangseinrichtung mit der Speichereinrichtung gespeicherte Daten berührungslos übertragbar sind.

Ein Signal- oder Datenaustausch zwischen der ersten und zweiten Sende- und Empfangseinrichtung wird besonders einfach, wenn diese ausgebildet sind zum Senden und Empfangen elektromagnetischer Wellen. Als Frequenzen kommen hier Frequenzen in Bereichen zwischen 30 und 500 kHz, 3 bis 30 MHz, im 433 MHz-Band, zwischen 850 und 950 MHz sowie Mikrowellenfrequenzen in einem Bereich von 2,4 bis 2,5 GHz infrage.

Grundsätzlich wäre es denkbar, die erste und die zweite Sende- und Empfangseinrichtung elektrisch leitend miteinander zu verbinden. Um insbesondere die zweite Sende- und Empfangseinrichtung auch für andere Zwecke nutzen zu können, ist es vorteilhaft, wenn die erste und die zweite Sende- und Empfangseinrichtung berührungslos miteinander gekoppelt sind. So kann insbesondere bei unterschiedlicher Bestromung der zweiten Sende- und Empfangseinrichtung verhindert werden, dass Schädigungen an der Antriebseinheit auftreten können.

Eine besonders einfache Form der Kopplung zwischen der ersten und zweiten Sende- und Empfangseinrichtung ist eine induktive Kopplung. Grundsätzlich wäre es auch möglich, die Sende- und Empfangseinrichtungen kapazitiv miteinander zu koppeln.

Günstigerweise ist die chirurgische Antriebseinheit mit einer Motoridentifikationseinrichtung zum Identifizieren der Art oder des Typs der chirurgischen Antriebseinheit und/oder des Motors ausgestattet. Die Motoridentifikationseinrichtung ermöglicht es, beispielsweise in der Speichereinrichtung der Antriebseinheit hinterlegte Daten betreffend die Art oder den Typ der Antriebseinheit und/oder des Motors vor deren Betrieb abzufragen und beispielsweise auf eine Steuer- und/oder Regelungseinrichtung zu übertragen.

Besonders einfach wird der Aufbau der Motoridentifikationseinrichtung sowie der Antriebseinheit, wenn die Motoridentifikationseinrichtung die Speichereinrichtung umfasst. So kann mit einer minimalen Zahl von Bauteilen eine Antriebseinheit ausgebildet werden.

Noch kompakter und einfacher lässt sich die chirurgische Antriebseinheit ausbilden, wenn die Motoridentifikationseinrichtung die erste Sende- und Empfangseinrichtung umfasst. So lässt sich quasi durch Einbau eines einzigen Bauteils nicht nur die Speichereinrichtung, sondern optional auch die erste Sende- und Empfangseinrichtung, in einem Montageschritt in die Antriebseinheit einbauen.

Besonders einfach und kostengünstig herzustellen wird die chirurgische Antriebseinheit, wenn die Motoridentifikationseinrichtung in Form eines RFID (Radio Frequency Identification)-Bauelements ausgebildet ist. Derartige Bauelemente, die auch als RFID-Chips oder Transponder-Chips bezeichnet werden, sind in unterschiedlichen Bauformen und Größen erhältlich und eignen sich zum Einbau als Baugruppe, welche mehrere Unterfunktionen realisieren kann. Insbesondere kann ein RFID-Chip eine Sende- und Empfangseinrichtung sowie eine Speichereinrichtung umfassen.

Um eine besonders gute Ankopplung für einen guten Austausch von Signalen und Daten zu haben, ist es vorteilhaft, wenn die Motoridentifikationseinrichtung räumlich in der Nähe der zweiten Sende- und Empfangseinrichtung angeordnet ist. Vorzugsweise wird die Anordnung der von der Motoridentifikationseinrichtung umfassten ersten Sende- und Empfangseinrichtung konstruktiv so gewählt, dass die erste und zweite Sende- und Empfangseinrichtung optimiert miteinander wechsel- und zusammenwirken können.

Um keine zusätzliche Energiequelle für die Motoridentifikationseinrichtung zu benötigen, ist es günstig, wenn diese in Form einer passiven Motoridentifikationseinrichtung ausgebildet ist. Selbstverständlich könnte auch eine aktive Motoridentifikationseinrichtung vorgesehen werden, welche eine Energieversorgung zum Betreiben ihrer Untereinheiten benötigt. Passive Motoridentifikationseinrichtungen haben den Vorteil, dass sie eine zu ihrem Betrieb erforderliche Energie aus einem hochfrequenten elektromagnetischen Wechselfeld entnehmen können, welches durch die zweite Sende- und Empfangseinrichtung erzeugt wird.

Günstigerweise sind mit der ersten und/oder zweiten Sende- und Empfangseinrichtung elektromagnetische Hochfrequenzfelder geringer Reichweite erzeugbar. Somit können insbesondere kurze Abstände zwischen den Sende- und Empfangseinrichtungen ohne Schwierigkeiten und mit hoher Präzision überbrückt werden.

Vorteilhafterweise umfasst die Speichereinrichtung einen nicht-flüchtigen Speicher. So wird sichergestellt, dass die im Speicher enthaltenen Daten dauerhaft erhalten bleiben.

Um zu verhindern, dass von einem Benutzer die in der Speichereinrichtung enthaltenen Daten versehentlich gelöscht oder überschrieben werden können, ist es vorteilhaft, wenn die Speichereinrichtung einen nicht-beschreibbaren Speicher umfasst. Dadurch ist jegliche Manipulation der Speichereinrichtung, um die darin vom Hersteller gespeicherten Daten zu ändern, ausgeschlossen.

Günstig ist es, wenn die Speichereinrichtung in Form eines Nur-Lese-Speicher (ROM) ausgebildet ist. Ein solcher Speicher kann beispielsweise nur einmalig beschrieben werden, jedoch beliebig oft ausgelesen. So kann beispielsweise ein Hersteller jeder Antriebseinheit individuelle Daten zu deren Charakterisierung zuordnen, beispielsweise den Motortyp, eine Seriennummer oder auch eine Typ- oder Seriennummer der Antriebseinheit insgesamt.

Vorzugsweise umfasst der Motor drei Motorwicklungen. Dies ermöglicht es, herkömmliche Motoren zur Ausbildung der Antriebseinheit zu nutzen.

Gemäß einer bevorzugten Ausführungsform der Erfindung kann ferner vorgesehen sein, dass die Antriebseinheit eine Motorsperreinrichtung zum Sperren eines Betriebs des Motors umfasst. Diese Weiterbildung ist insbesondere auch bei einer chirurgischen Antriebseinheit der eingangs beschriebenen Art möglich. Die Motorsperreinrichtung gestattet es, gezielt festzulegen, ob und wann ein Motor der Antriebseinheit überhaupt betrieben, insbesondere bestromt, werden kann. Erst durch eine entsprechende Aktivierung oder Deaktivierung der Motorsperreinrichtung kann beispielsweise der Motor entsperrt und betrieben werden.

Besonders einfach wird der Bau der Motorsperreinrichtung, wenn diese ein mit zwei Motoranschlusskontakten verbundenes Schaltelement umfasst. Vor dem eigentlichen Betrieb des Motors kann dann beispielsweise über zwei Motoranschlusskontakte, also beispielsweise über zwei Wicklungskontakte, abgefragt werden, ob das Schaltelement geöffnet oder geschlossen ist. Je nach Schaltstellung des Schaltelements kann dann beispielsweise in einer Steuer- und/oder Regelungseinrichtung detektiert werden, ob die Motorsperreinrichtung eine Sperr- oder Freigabestellung einnimmt, in welcher der Motorbetrieb gesperrt beziehungsweise freigegeben ist.

Um die Abfrage der Motorsperreinrichtung zu vereinfachen, insbesondere ob diese eine Sperr- oder Freigabestellung einnimmt, ist es günstig, wenn die Motorsperreinrichtung einen mit dem Schaltelement in Serie zwischen zwei Motoranschlusskontakte geschalteten frequenzabhängigen Widerstand umfasst. Beispielsweise in einem Abfragebetrieb der Steuer- und/oder Regelungseinrichtung kann so über die zwei Motoranschlusskontakte ein Hochfrequenzstrom geleitet werden. Ist das Schaltelement geschlossen, dann kann in Abhängigkeit der Art des frequenzabhängigen Widerstands der Hochfrequenzstrom durchgeleitet werden oder nicht.

Besonders einfach wird der Aufbau der Motorsperreinrichtung, wenn der frequenzabhängige Widerstand ein Kondensator ist. Dieser leitet einen Hochfrequenzstrom, so dass ein Stromfluss über die zwei Motoranschlusskontakte detektiert werden kann, wenn das Schaltelement geschlossen ist.

Vorzugsweise umfasst die chirurgische Antriebseinheit einen Temperatursensor. Dieser gestattet es, eine Temperatur der Antriebseinheit zu bestimmen. Beispielsweise kann dies eine Temperatur der Antriebseinheit vor oder nach Inbetriebnahme sein. Denkbar wäre auch eine Temperaturbestimmung während eines Betriebs, um so ein Betriebstemperaturprofil zu erstellen.

Der Aufbau der Antriebseinheit wird besonders einfach, wenn die Motoridentifikationseinrichtung den Temperatursensor umfasst.

Um Temperaturdaten von der Antriebseinheit auslesen zu können, ist es günstig, wenn zwischen der ersten und zweiten Sende- und Empfangseinrichtung mit dem Temperatursensor erfasste Temperaturdaten berührungslos austauschbar sind. Beispielsweise kann die erste Sende- und Empfangseinrichtung mit dem Temperatursensor direkt oder indirekt über eine entsprechende Schaltung verbunden sein.

Um beispielsweise nach einem Betrieb der Antriebseinheit ein Temperaturprofil während des Betriebs der Antriebseinheit aufnehmen zu können, ist es vorteilhaft, wenn der Temperatursensor einen Temperaturdatenspeicher umfasst.

Bei einem erfindungsgemäß weitergebildeten chirurgischen Instrument ist es günstig, wenn mindestens eine der mindestens zwei chirurgischen Antriebseinheiten eine der oben beschriebenen chirurgischen Antriebseinheiten ist. Das Instrument weist dann die oben im Zusammenhang mit den vorteilhaften Ausgestaltungen der Antriebseinheit beschriebenen Vorteile auf.

Des Weiteren ist es bei einem erfindungsgemäß weitergebildeten Antriebssystem günstig, wenn mindestens eine der mindestens zwei chirurgischen Antriebseinheiten eine der oben beschriebenen chirurgischen Antriebseinheiten ist. Folglich weist auch das chirurgische Antriebssystem die im Zusammenhang mit den oben beschriebenen vorteilhaften Ausführungsformen chirurgischer Antriebseinheiten erläuterten Vorteile auf.

Günstigerweise umfassen bei einem chirurgischen Antriebssystem die mindestens zwei chirurgischen Antriebseinheiten unterschiedliche Motortypen. Sie können dann ohne Weiteres in der oben im Detail beschriebenen Weise, insbesondere automatisch, identifiziert werden.

Für einen Nutzer wird der Aufbau des Antriebssystems besonders einfach handhabbar, wenn die Steuer- und/oder Regelungseinrichtung in Form eines Motorsteuergerätes ausgebildet ist. Dieses kann insbesondere entsprechende Schnittstellen zum Verbinden mit Anschlussleitungen oder Anschlusskabeln umfassen, die an ihrem anderen Ende mit einer Antriebseinheit oder einem Motor des Antriebssystems, insbesondere mit den Motorwicklungen, verbindbar oder verbunden sind. Das Motorsteuergerät kann insbesondere auch eine Anzeigevorrichtung aufweisen, auf der vorgegebene und aktuelle Betriebsdaten und Parameter des Systems angezeigt werden können.

Um die mindestens zwei Antriebseinheiten mit der Steuer- und/oder Regelungseinrichtung verbinden zu können, ist es vorteilhaft, wenn Anschlusskabel, also mindestens ein Anschlusskabel, vorgesehen sind, mit denen die Antriebseinheiten mit der Steuer- und/oder Regelungseinrichtung verbindbar sind. Die Anschlusskabel können dauerhaft mit der Antriebseinheit oder einem Motor verbunden oder mit diesen lösbar verbindbar sein.

Vorteilhafterweise umfassen die Anschlusskabel jeweils nur so viele Leitungen, wie der mit ihnen verbundene oder verbindbare Motor Motorwicklungen umfasst. So wird der Aufbau der Anschlusskabel besonders einfach, denn es müssen keine Steuer- und/oder Datenleitungen vorgesehen werden. Optional können selbstverständlich zusätzlich Steuer- und/oder Datenleitungen vorgesehen sein.

Vorzugsweise ist die Steuer- und/oder Regelungseinrichtung des Antriebssystems derart ausgebildet, dass in einem ersten Abfragemodus detektierbar ist, ob eine Antriebseinheit mit der Steuer- und/oder Regelungseinrichtung verbunden ist. Bevor der eigentliche Betrieb der Antriebseinheit aufgenommen wird, beispielsweise durch Bestromen der Motorwicklungen, kann so im ersten Abfragemodus detektiert werden, ob überhaupt eine Antriebseinheit mit der Steuer- und/oder Regelungseinrichtung verbunden ist. Hier kann quasi in einem Stand-By-Betrieb permanent abgefragt werden, ob ein Motor kontaktiert ist.

Des Weiteren kann es vorteilhaft sein, wenn die Steuer- und/oder Regelungseinrichtung derart ausgebildet ist, dass dann, wenn eine Antriebseinheit mit der Steuer- und/oder Regelungseinrichtung verbunden ist, in einem zweiten Abfragemodus die in der Speichereinrichtung hinterlegten Daten unter Verwendung der ersten und zweiten Sende- und Empfangseinrichtung auslesbar und an die Steuer- und/oder Regelungseinrichtung übertragbar sind. Wird bei der Abfrage der Antriebseinheit durch das Steuergerät festgestellt, dass eine Antriebseinheit kontaktiert ist, wird somit im zweiten Abfragemodus beispielsweise der Motortyp abgefragt und an die Steuer- und/oder Regelungseinrichtung übermittelt.

Des Weiteren kann es günstig sein, wenn die Steuer- und/oder Regelungseinrichtung derart ausgebildet ist, dass dann, wenn eine Antriebseinheit mit der Steuer- und/oder Regelungseinrichtung verbunden ist, in einem dritten Abfragemodus durch Leiten eines hochfrequenten Stromes über die mit der Motorsperreinrichtung verbundenen Anschlusskontakte detektierbar ist, ob das Schaltelement geöffnet oder geschlossen ist. Auf diese Weise kann festgestellt werden, ob die Motorsperreinrichtung eine Freigabestellung, in welcher der Motor betrieben werden kann, oder eine Sperrstellung, in welcher der Motor nicht betrieben werden kann, einnimmt. In Abhängigkeit des Abfrageergebnisses kann die Steuer- und/oder Regelungseinrichtung eine Bestromung der Motorwicklungen gezielt zulassen oder auch nicht.

Um Fehlfunktionen des Antriebssystems zu vermeiden, ist es vorteilhaft, wenn die Steuer- und/oder Regelungseinrichtung derart ausgebildet ist, dass der Motor nur bestrombar ist, wenn im dritten Abfragemodus detektiert wurde, dass das Schaltelement geschlossen ist. Ist beispielsweise das Schaltelement so ausgebildet, dass eine Bedienperson anhand einer Stellung des Schaltelements oder eines Betätigungselements desselben erkennen kann, ob die Motorsperreinrichtung die Sperrstellung oder die Freigabestellung einnimmt, kann so ein sicherer Betrieb des Antriebssystems und seiner Antriebseinheiten sichergestellt werden.

Um Daten auf einfache Weise aus der Speichereinrichtung der Antriebseinheit auslesen zu können, ist es vorteilhaft, wenn die Steuer- und/oder Regelungseinrichtung derart ausgebildet ist, dass die in der Speichereinrichtung der Antriebseinheit hinterlegten Daten unter Verwendung der ersten und zweiten Sende- und Empfangseinrichtung auslesbar und an die Steuer- und/oder Regelungseinrichtung übertragbar sind.

Des Weiteren kann es günstig sein, wenn die Steuer- und/oder Regelungseinrichtung derart ausgebildet ist, dass mit dem Temperatursensor bestimmte Temperaturdaten unter Verwendung der ersten und zweiten Sende- und Empfangseinrichtung auslesbar und an die Steuer- und/oder Regelungseinrichtung übertragbar sind. Auf diese Weise ist es möglich, Temperaturdaten auszulesen und zur weiteren Verarbeitung an die Steuer- und/oder Regelungseinrichtung zu übermitteln. Insbesondere kann so nach einem Betrieb der Antriebseinheit festgestellt werden, ob während des letzten Betriebsintervalls eine Überhitzung des Motors erfolgt ist. Gegebenenfalls kann dann eine weitere Bestromung der Antriebseinheit unterbunden werden, um Fehlfunktionen und gegebenenfalls eine Zerstörung der Antriebseinheit zu vermeiden.

Vorteilhafterweise ist die Steuer- und/oder Regelungseinrichtung derart ausgebildet, dass die Temperaturdaten im zweiten Abfragemodus übertragbar sind. Dies bedeutet, dass sie vorzugsweise nur dann übertragbar sind, wenn die Antriebseinheit mit der Steuer- und/oder Regelungseinrichtung verbunden ist, jedoch nicht betrieben wird, also ihre Motorwicklungen unbestromt sind.

Zur Erhöhung der Betriebssicherheit des Antriebssystems kann es günstig sein, wenn die Steuer- und/oder Regelungseinrichtung derart ausgebildet ist, dass Betriebsparameter zum Betreiben einer mit der Steuer- und/oder Regelungseinrichtung verbundenen Antriebseinheit in Abhängigkeit der aus der Speichereinrichtung ausgelesenen Daten automatisch einstellbar sind. Mit anderen Worten bedeutet dies, dass beispielsweise ein Maximalstrom und damit eine Maximaldrehzahl der Antriebseinheit in Abhängigkeit des mit der Steuer- und/oder Regelungseinrichtung verbundenen Motors automatisch begrenzt werden können. Die dem Motortyp zuzuordnenden Betriebsparameter können beispielsweise in einem Speicher der Steuer- und/oder Regelungseinrichtung hinterlegt sein. So lassen sich auch nachträglich, ohne die Antriebseinheiten ändern zu müssen, Betriebsparameter für bestimmte Motoren auf den neuesten Stand bringen, beispielsweise mit neuen Programmversionen einer Steuer- und/oder Regelungssoftware der Steuer- und/oder Regelungseinrichtung.

Die nachfolgende Beschreibung bevorzugter Ausführungsformen der Erfindung dient im Zusammenhang mit der Zeichnung der näheren Erläuterung. Es zeigen:
- Figur 1:: eine schematische Übersichtsdarstellung eines chirurgischen Antriebssystems;
- Figur 2:: eine schematische Darstellung eines chirurgischen Instruments sowie einer Steuer- und/oder Regelungseinrichtung eines chirurgischen Antriebssystems;

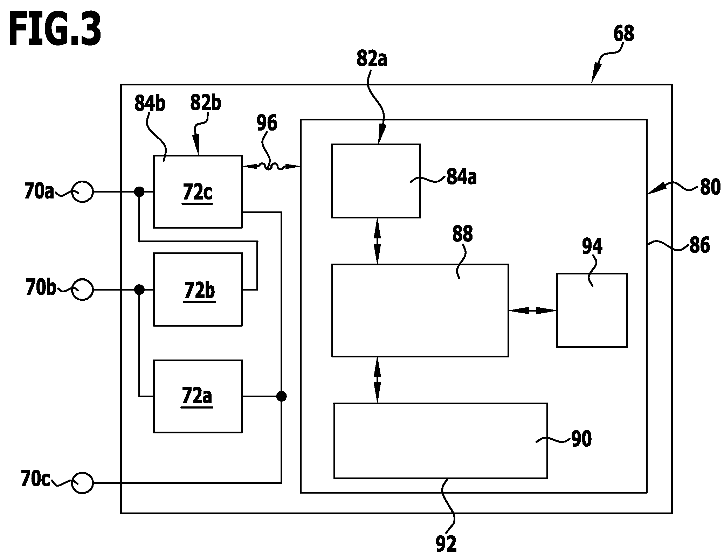

- Figur 3:: eine schematische Darstellung eines Motors einer Antriebseinheit mit einer Motoridentifikationseinrichtung;

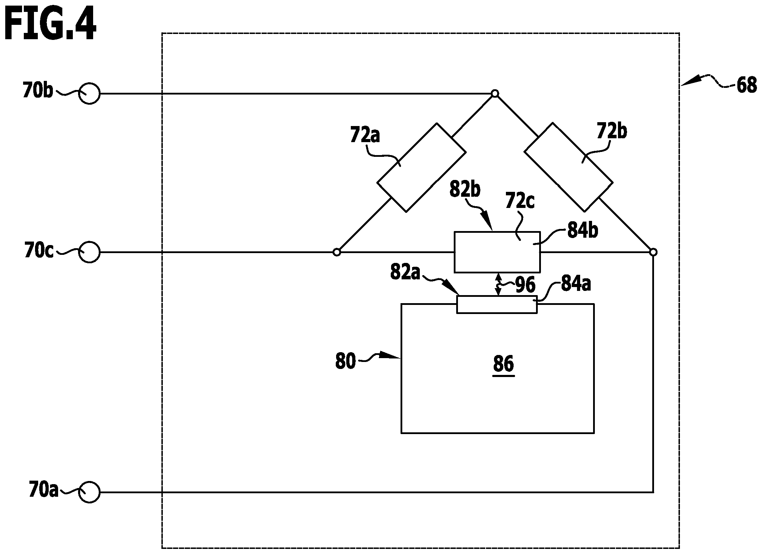

- Figur 4:: eine Schaltplanskizze eines Motors einer Antriebseinheit mit Motoridentifikationseinrichtung; und

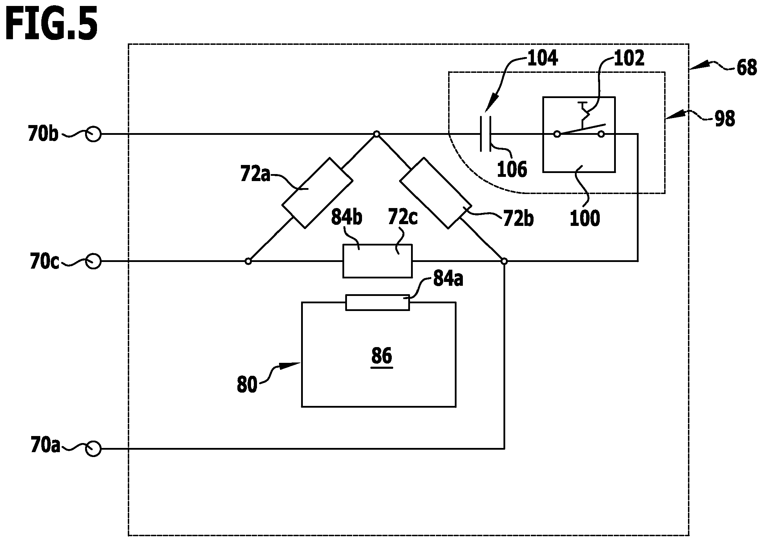

- Figur 5:: eine Schaltskizze analog Figur 4 mit zusätzlicher Motorsperreinrichtung.

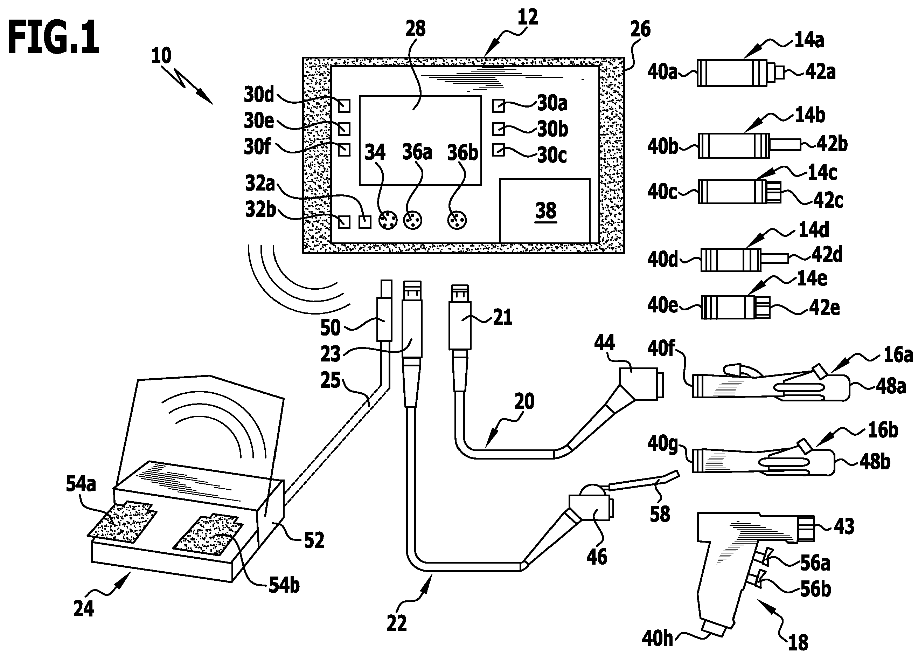

In Figur 1 ist schematisch ein insgesamt mit dem Bezugszeichen 10 versehenes chirurgisches Antriebssystem dargestellt, umfassend eine Steuer- und/oder Regelungseinrichtung in Form eines Steuergeräts 12, fünf Antriebseinheiten 14a bis 14e, zwei, ebenfalls Antriebseinheiten bildende Shaverhandstücke 16a und 16b, ein eine weitere Antriebseinheit bildendes Pistolenhandstück 18, zwei Anschlusskabel 20 und 22 sowie eine Fußsteuerung 24.

Das Steuergerät 12 umfasst einen in einem Gehäuse 26 angeordneten flachen Bildschirm 28 in Form eines Touchscreens. Zu beiden Seiten des Bildschirms 28 sind je drei Bedienelemente 30a bis 30c beziehungsweise 30d bis 30f angeordnet.

Zwei Schalter 32a und 32b sind unterhalb des Bildschirms 28 auf einer Linie angeordnet mit einer Anschlussbuchse 34 zum Anschluss der Fußsteuerung 24 über ein optionales Anschlusskabel 25 und mit zwei Anschlussbuchsen 36a und 36b zum Anschluss der Anschlusskabel 20 und 22, mit denen die Antriebseinheiten mit dem Steuergerät 12 verbunden werden können. Optional kann außerdem ein Anschluss 38 für ein Fluidsystem zur Zufuhr und Abfuhr von Fluiden aus einem Operationsbereich vorgesehen sein, beispielsweise auch zur Versorgung von Spül- oder Absaugkanälen an mit den Antriebseinheiten 14, den Shaverhandstücken 16 oder dem Pistolenhandstück 18 verbindbaren, nicht dargestellten Handstücken oder Werkzeugen, mit welchen zusammen die Antriebseinheiten chirurgische Instrumente des Antriebssystems 10 bilden.

Die Antriebseinheiten 14a bis 14e umfassen jeweils eine Kabelkupplung 40a bis 40e, die mit einem Kupplungsstück 44 des Anschlusskabels 20 oder einem Kupplungsstück 46 des Anschlusskabels 22 beliebig verbindbar sind. Ebenso weisen die beiden Shaverhandstücke 16a und 16b sowie das Pistolenhandstück 18 jeweils eine Kabelkupplung 40f, 40g beziehungsweise 40h auf, die mit einem der beiden Kupplungsstücke 44 oder 46 verbindbar sind.

An ihrem jeweils anderen Ende sind die Antriebseinheiten 14a bis 14e mit Handstück- oder Werkzeugkupplungen 42a bis 42e ausgestattet, auf die nicht dargestellte Handstücke, beispielsweise Bohrerhandstücke, Sägehandstücke oder dergleichen angekuppelt werden können, die durch die Antriebseinheiten 14a bis 14e angetrieben werden können. Je nach Ausgestaltung können die Antriebseinheiten 14a bis 14e auch direkt mit nicht dargestellten Werkzeugen, wie beispielsweise Bohrern oder Sägeblättern, bestückt werden zur Ausbildung chirurgischer Instrumente.

Die Antriebseinheiten sind vorzugsweise sensorlos ausgebildet, das heißt sie weisen keine Sensoren auf, um eine Drehzahl der Antriebseinheit während des Betriebs zu bestimmen. Die Antriebseinheiten des Antriebssystems 10 unterscheiden sich nicht nur, wie in Figur 1 schematisch dargestellt, äußerlich, sondern auch hinsichtlich ihres inneren Aufbaus. Dies bedeutet, dass die in den Antriebseinheiten verbauten Motoren unterschiedlichen Typs sein und sich beispielsweise in ihren Kenngrößen, wie zum Beispiel Minimaldrehzahl, Maximaldrehzahl, Maximalstrom und Maximaldrehmoment, unterscheiden können. Zudem können, wie bei den beiden Shaverhandstücken 16a und 16b, Getriebe integriert sein, welche optional auch in an die Antriebseinheiten 14 sowie an das Pistolenhandstück 18 ankoppelbare Handstücke integriert sein können. Die Handstücke können je nach Ausgestaltung auch selbst zusätzlich mit unterschiedlichen Instrumentenspitzen in Form chirurgischer Werkzeuge bestückt werden.

Des Weiteren umfassen die Shaverhandstücke 16a und 16b jeweils eine Shaverkupplung 48a beziehungsweise 48b zum Anschluss eines Shavers, beispielsweise zur Anwendung in der Arthroskopie.

Die Anschlusskabel 20 und 22 sind zum Verbinden mit dem Steuergerät mit Kupplungen 21 und 23 versehen, über die sie mit den Anschlussbuchsen 36a und 36b verbindbar sind.

Die Fußsteuerung 24 steht über eine drahtlose Datenübertragungseinrichtung mit dem Steuergerät 12 in Verbindung, beispielsweise über ein Infrarot- oder Funkübertragungssystem. Optional ist auch eine Verbindung der Fußsteuerung 24 über ein mit der Anschlussbuchse 34 verbindbares Kupplungsstück 50 des Anschlusskabels 25 möglich. An einem Gehäuse 52 der Fußsteuerung 24 sind zwei fußbetätigbare Schalter 54a und 54b angeordnet, über die insbesondere ein Links- beziehungsweise Rechtslauf der Antriebseinheiten geregelt werden kann.

Das Pistolenhandstück 18 ist mit zwei Gebern 56 ausgestattet, wobei der Geber 56a beispielsweise zur Aktivierung eines Motorrechtslaufes, der Geber 56b zur Aktivierung eines Motorlinkslaufes vorgesehen sein können.

Die Anschlusskabel 20 und 22 unterscheiden sich dadurch, dass am Anschlusskabel 22, anders als am Anschlusskabel 20, ein Betätigungshebel 58 vorgesehen ist, mit dem eine Bedienperson einen Motorbetrieb einer Antriebseinheit 14, eines Shaverhandstücks 16 oder des Pistolenhandstücks 18 aktivieren kann.

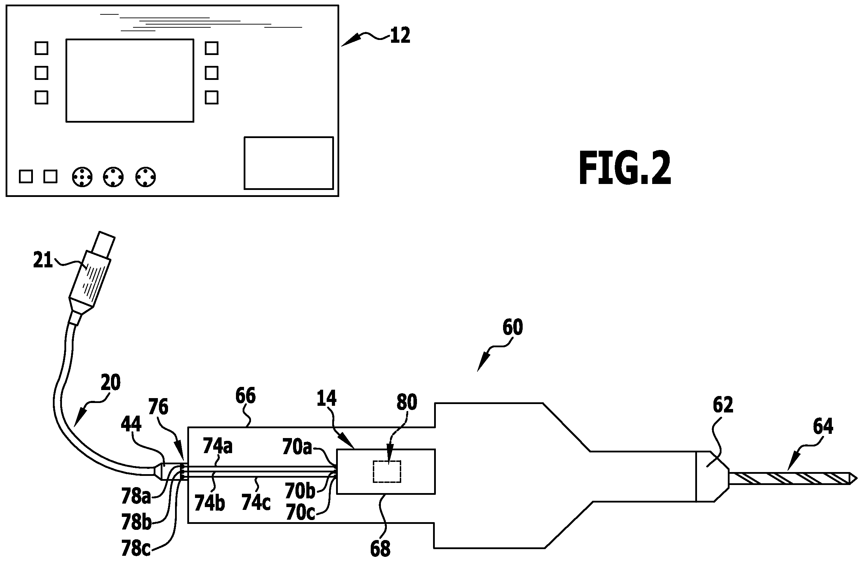

In Figur 2 ist schematisch der Aufbau eines chirurgischen Instruments 60 dargestellt. Es umfasst eine Antriebseinheit 14, beispielsweise eine Antriebseinheit 14a bis 14e, sowie ein mit einer distalseitigen Kupplung 62 desselben verbindbares Werkzeug 64, zum Beispiel in Form eines in Figur 2 dargestellten Bohrers.

In einem Gehäuse 66 der Antriebseinheit 14 ist ein Motor 68 angeordnet, welcher drei Anschlusskontakte 70a, 70b und 70c aufweist, die jeweils mit zwei der insgesamt drei Motorwicklungen 72a, 72b und 72c verbunden sind. Der Anschlusskontakt 70a ist mit den Motorwicklungen 72b und 72c verbunden, der Anschlusskontakt 70b mit den Motorwicklungen 72a und 72b und der Anschlusskontakt 70c mit den Motorwicklungen 72a und 72c.

Die Anschlusskontakte 70a, 70b und 70c sind mittels Verbindungsleitungen 74a, 74b und 74c mit einem proximalseitig angeordneten Kupplungselement 76 verbunden, und zwar mit dessen Verbindungskontakten 78a, 78b und 78c. Das Kupplungselement 76 ist zum Verbinden mit dem Kupplungsstück 44 des Anschlusskabels 20 korrespondierend zu diesem ausgebildet.

Das Anschlusskabel 20 selbst ist ein dreiadriges Anschlusskabel. Bei dem in Figur 2 dargestellten Ausführungsbeispiel sind keine Steuer- oder Datenleitungen am Anschlusskabel 20 oder an der Antriebseinheit 14 vorgesehen.

In den Motor 68 ist ferner eine Motoridentifikationseinrichtung 80 integriert. Diese wiederum umfasst eine erste Sende- und Empfangseinrichtung 82a in Form einer Antenne 84a. Die Antenne 84a ist Teil eines RFID-Chips 86, der ferner eine elektronische Schaltung 88 umfasst, welche mit der Antenne 84a sowie einer Speichereinrichtung 90 in Form eines elektronischen Datenspeichers verschaltet ist. Vorzugsweise handelt es sich bei dem Datenspeicher 92 um einen ROM-Speicher, welcher nicht-flüchtig und nicht-beschreibbar ist. Im Datenspeicher 92 sind insbesondere Daten abgelegt, die den Motortyp 68 und/oder den Typ der Antriebseinheit 14 charakterisieren. Des Weiteren kann die Motoridentifikationseinrichtung 80 optional einen Temperatursensor 94 umfassen, welcher ebenfalls mit der Schaltung 88 verschaltet ist.

Zur Kommunikation mit der ersten Sende- und Empfangseinrichtung 82a dient eine Sende- und Empfangseinrichtung 82b in Form einer Antenne 84b, die durch die Motorwicklung 72c gebildet wird. Die Antenne 84b ist folglich elektrisch leitend mit den Anschlusskontakten 70a und 70c verbunden.

Die Sende- und Empfangseinrichtungen 82a und 82b sind räumlich dicht beieinander angeordnet, so dass eine optimale induktive Koppelung gewährleistet ist. Ein Informations- beziehungsweise Datenaustausch zwischen den Sende- und Empfangseinrichtungen 82a und 82b erfolgt berührungslos mittels elektromagnetischer Wellen 96.

Die Funktionsweise des Antriebssystems 10 wird nachfolgend kurz erläutert. Das Steuergerät 12 prüft in einem ersten Abfragemodus, welcher auch als Standby-Betrieb bezeichnet werden kann, ob eine Antriebseinheit 14 mit dem Steuergerät 12 verbunden ist. Es wird dabei insbesondere geprüft, ob mindestens eine der Motorwicklungen 72a, 72b oder 72c über das Anschlusskabel 20 mit dem Steuergerät 12 verbunden ist. Solange kein Motor 68, das heißt keine der Motorwicklungen 72a, 72b oder 72c erkannt wird, wird weiter abgefragt.

Sobald das Steuergerät 12 eine mit ihm kontaktierte Motorwicklung 72a, 72b oder 72c erkennt, wird über die Motorwicklung 72c in einem zweiten Abfragemodus durch entsprechende Bestromung der Anschlusskontakte 70a und 70c der RFID-Chip 86, der auch als Transponder bezeichnet werden kann, abgefragt, das heißt, die im Datenspeicher 92 hinterlegten Daten ausgelesen und durch Kommunikation der Sende- und Empfangseinrichtung 82a und 82b ausgelesen und in das Steuergerät 12 übertragen. Im Steuergerät 12 können dann entsprechend dem detektierten Motortyp aus einem nicht dargestellten Speicher hinterlegte Betriebsinformationen oder Parameter ausgelesen und das Steuergerät 12 entsprechend eingestellt werden. Nach der Detektion des Motortyps kann dann insbesondere der Betrieb der Antriebseinheit 14 aufgenommen werden.

Optional kann die Antriebseinheit 14 auch mit einer in den Motor 68 integrierten Motorsperreinrichtung 98 ausgestattet sein. Sie umfasst ein Schaltelement 100 in Form eines selbsthaltenden Tasters, welcher ein mindestens teilweise aus dem Gehäuse 66 herausragendes Betätigungselement 102 umfasst. Mit dem Schaltelement 100 ist ein frequenzabhängiger Widerstand 104 in Form eines Kondensators 106 in Serie zwischen den Anschlusskontakten 70a und 70b geschaltet.

Der Motor 68 der Antriebseinheit 14 kann wie folgt gesperrt werden: Im Standby-Betrieb (erster Abfragemodus) des Steuergeräts 12 kann auch ein Hochfrequenzstrom über die Anschlusskontakte 70a und 70b geleitet werden. Ist das Schaltelement 100 geschlossen, kann ein hochfrequenter Strom zwischen den Anschlusskontakten 70a und 70b über den dann leitenden Kondensator 106 fließen. Ist das Schaltelement 100 geöffnet, ist ein Stromfluss nicht möglich. So lässt sich eine intelligente Motorsperrfunktion integrieren. Die Abfrage der Antriebseinheit 14, ob die Motorsperreinrichtung 98 eine Sperrstellung definiert, in welcher der Motor 68 nicht bestromt werden soll, oder eine Freigabestellung, in welcher der Motor 68 bestrombar sein soll, kann in einem dritten Abfragemodus erfolgen, und zwar vor oder nach der Abfrage des Motortyps. Die detektierte Stellung der Motorsperreinrichtung 98 wird im Steuergerät 12 übernommen und in Abhängigkeit der detektierten Stellung der Betrieb des Motors 68 entsprechend einer Drehzahlanforderung, beispielsweise über die Fußsteuerung 24, erfolgen oder auch nicht. Wird die Drehzahlanforderung wieder auf Null zurückgefahren, wird der Motor 68 bis zum Stillstand abgebremst und die Abfragemodi werden in der oben beschriebenen Weise wieder aktiviert.

Der Temperatursensor 94 kann genutzt werden, um unter Verwendung eines im Steuergerät 12 hinterlegten Softwaremodells die während des Betriebs im Motor 68 erreichte Betriebstemperatur zu berechnen. Das Softwaremodell basiert insbesondere auf der Annahme, dass die Umgebungstemperatur einen bestimmten Wert T_{U} aufweist, beispielsweise 25° C, und dass die Motortemperatur T_{M} vor dem Start der ersten Motoraktivierung der Umgebungstemperatur T_{U} entspricht, also beispielsweise ebenfalls 25° C. Durch den bekannten konstruktiven Aufbau des Motors 68 ist dessen Wärmekapazität bekannt. Des Weiteren ist mit dem Steuergerät 12 die in den Motor 68 während des Betriebs transportierte Strommenge pro Zeiteinheit bekannt. Mit einer im Steuergerät 12 integrierten Rechnereinheit, einer sogenannten DSP-Steuerung, ist es möglich, die Motorerwärmungskurven sowie in den Ruhephasen die Motorabkühlkurven nachzubilden. Ein Zurücksetzen des Softwaremodells erfolgt durch Trennen des Motors 68 vom Motorsteuergerät 12.

Die Genauigkeit der Motortemperaturerfassung kann durch den Temperatursensor 94 verbessert werden. Vor Beginn der ersten Aktivierung des Motors 68, nach jedem Kontaktieren des Motors 68 mit dem Steuergerät 12 sowie auch in den Ruhephasen zwischen zwei Aktivierungszyklen wird die Motortemperatur unter Verwendung der Sende- und Empfangseinrichtungen 82a und 82b ausgelesen und die durch das Softwaremodell berechnete Motortemperatur entsprechend korrigiert. Es ist damit möglich, die Motortemperatur nicht nur sehr viel präziser zu erfassen als ohne Temperatursensor, sondern es ist ferner nun auch möglich, bereits vor einer ersten Aktivierung einen zu diesem Zeitpunkt schon überhitzen Motor 68 vor einer nicht akzeptablen weiteren Aktivierung durch entsprechendes Sperren durch das Steuergerät 12 zu hindern. Optional könnte die Aufgabe der Temperaturerfassung auch durch einen weiteren RFID-Chip übernommen werden, welcher in den Motor 68 beziehungsweise die Antriebseinheit 14 integriert werden könnte.

## Patentansprüche

1. Chirurgische Antriebseinheit (14) eines chirurgischen Instruments (60), welche einen Motor (68) mit mindestens zwei Motorwicklungen (72a, 72b, 72c) und eine Speichereinrichtung (90) zur Speicherung von die Antriebseinheit (14) und/oder den Motor (68) charakterisierenden Daten umfasst, wobei die Antriebseinheit (14) eine erste Sende- und Empfangseinrichtung (82a), welche mit der Speichereinrichtung (90) verbunden ist, und eine zweite Sende- und Empfangseinrichtung (82b), welche mit mindestens zwei Anschlusskontakten (70a, 70c) des Motors (68) verbunden ist, umfasst und wobei die erste und die zweite Sende- und Empfangseinrichtung (82a, 82b) derart ausgebildet sind und zusammenwirken, dass Daten berührungslos zwischen ihnen übertragbar sind, **dadurch gekennzeichnet, dass** eine (72c) der mindestens zwei Motorwicklungen (72a, 72b, 72c) die zweite Sende- und Empfangseinrichtung (82b) bildet.

2. Chirurgische Antriebseinheit nach Anspruch 1, **dadurch gekennzeichnet, dass** zwischen der ersten und zweiten Sende- und Empfangseinrichtung (82a, 82b) in der Speichereinrichtung (90) gespeicherte Daten berührungslos übertragbar sind.

3. Chirurgische Antriebseinheit nach einem der voranstehenden Ansprüche, **dadurch gekennzeichnet, dass** die erste und die zweite Sende- und Empfangseinrichtung (82a, 82b) berührungslos miteinander gekoppelt sind.

4. Chirurgische Antriebseinheit nach einem der voranstehenden Ansprüche, **gekennzeichnet durch** eine Motoridentifikationseinrichtung (80) zum Identifizieren der Art oder des Typs der chirurgischen Antriebseinheit (14) und/oder des Motors (68).

5. Chirurgische Antriebseinheit nach Anspruch 4, **dadurch gekennzeichnet, dass** die Motoridentifikationseinrichtung (80) die erste Sende- und Empfangseinrichtung (82a) umfasst.

6. Chirurgische Antriebseinheit nach Anspruch 4 oder 5, **dadurch gekennzeichnet, dass** die Motoridentifikationseinrichtung (80) in Form eines RFID (Radio Frequency Identification)-Bauelements (86) ausgebildet ist.

7. Chirurgische Antriebseinheit nach einem der voranstehenden Ansprüche, **dadurch gekennzeichnet, dass** mit der ersten und/oder zweiten Sende- und Empfangseinrichtung (82a, 82b) elektromagnetische Hochfrequenzfelder geringer Reichweite erzeugbar sind.

8. Chirurgische Antriebseinheit nach einem der voranstehenden Ansprüche, **dadurch gekennzeichnet, dass** die Antriebseinheit (14) eine Motorsperreinrichtung (98) zum Sperren eines Betriebs des Motors (18) umfasst.

9. Chirurgisches Instrument (60) umfassend eine Antriebseinheit (14) und ein mit der Antriebseinheit (14) verbundenes oder verbindbares und von der Antriebseinheit (14) antreibbares chirurgisches Werkzeug (64), wobei die Antriebseinheit (14) einen Motor (68) mit mindestens zwei Motorwicklungen (72a, 72b, 72c) und eine Speichereinrichtung (90) zur Speicherung von die Antriebseinheit (14) und/oder den Motor (68) charakterisierenden Daten umfasst, wobei die Antriebseinheit (14) eine erste Sende- und Empfangseinrichtung (82a), welche mit der Speichereinrichtung (90) verbunden ist, und eine zweite Sende- und Empfangseinrichtung (82b), welche mit mindestens zwei Anschlusskontakten (70a, 70c) des Motors (68) verbunden ist, umfasst und wobei die erste und die zweite Sende- und Empfangseinrichtung (82a, 82b) derart ausgebildet sind und zusammenwirken, dass Daten berührungslos zwischen ihnen übertragbar sind, **dadurch gekennzeichnet, dass** eine (72c) der mindestens zwei Motorwicklungen (72a, 72b, 72c) die zweite Sende- und Empfangseinrichtung (82b) bildet.

10. Chirurgisches Instrument nach Anspruch 9, **dadurch gekennzeichnet, dass** die Antriebseinheit (14) eine chirurgische Antriebseinheit (14) nach einem der Ansprüche 2 bis 8 ist.

11. Chirurgisches Antriebssystem (10) umfassend mindestens eine Steuer- und/oder Regelungseinrichtung (12) und mindestens zwei mit dieser verbindbare und ansteuerbare chirurgische Antriebseinheiten (14) oder chirurgische Antriebseinheiten (14) umfassende chirurgische Instrumente (60), wobei mindestens eine der mindestens zwei chirurgischen Antriebseinheiten (14) einen Motor (68) mit mindestens zwei Motorwicklungen (72a, 72b, 72c) und eine Speichereinrichtung (90) zur Speicherung von die Antriebseinheit (14) und/oder den Motor (68) charakterisierenden Daten umfasst, wobei die Antriebseinheit (14) eine erste Sende- und Empfangseinrichtung (82a), welche mit der Speichereinrichtung (90) verbunden ist, und eine zweite Sende- und Empfangseinrichtung (82b), welche mit mindestens zwei Anschlusskontakten (70a, 70c) des Motors verbunden ist, umfasst und wobei die erste und die zweite Sende- und Empfangseinrichtung (82a, 82b) derart ausgebildet sind und zusammenwirken, dass Daten berührungslos zwischen Ihnen übertragbar sind, **dadurch gekennzeichnet, dass** eine (72c) der mindestens zwei Motorwicklungen (72a, 72b, 72c) die zweite Sende- und Empfangseinrichtung (82b) bildet.

12. Chirurgisches Antriebssystem nach Anspruch 11, **dadurch gekennzeichnet, dass** mindestens eine der mindestens zwei chirurgischen Antriebseinheiten (14) eine chirurgische Antriebseinheit (14) nach einem der Ansprüche 2 bis 8 ist.

13. Chirurgisches Antriebssystem nach Anspruch 11 oder 12, **dadurch gekennzeichnet, dass** die Steuer- und/oder Regelungseinrichtung (12) derart ausgebildet ist, dass in einem ersten Abfragemodus detektierbar ist, ob eine Antriebseinheit (14) mit der Steuer- und/oder Regelungseinrichtung (12) verbunden ist.

14. Chirurgisches Antriebssystem nach einem der Ansprüche 11 bis 13, **dadurch gekennzeichnet, dass** die Steuer- und/oder Regelungseinrichtung (12) derart ausgebildet ist, dass dann, wenn eine Antriebseinheit (14) mit der Steuer- und/oder Regelungseinrichtung (12) verbunden ist, in einem zweiten Abfragemodus die in der Speichereinrichtung (90) hinterlegten Daten unter Verwendung der ersten und zweiten Sende- und Empfangseinrichtung (82a, 82b) auslesbar und an die Steuer- und/oder Regelungseinrichtung (12) übertragbar sind.

15. Chirurgisches Antriebssystem nach einem der Ansprüche 11 bis 14, **dadurch gekennzeichnet, dass** die Steuer- und/oder Regelungseinrichtung (12) derart ausgebildet ist, dass dann, wenn eine Antriebseinheit (14) mit der Steuer- und/oder Regelungseinrichtung (12) verbunden ist, in einem dritten Abfragemodus durch Leiten eines hochfrequenten Stromes über die mit der Motorsperreinrichtung (98) verbundenen Anschlusskontakte (70a, 70b) detektierbar ist, ob das Schaltelement (100) geöffnet oder geschlossen ist.

## Claims

1. Surgical drive unit (14) of a surgical instrument (60), comprising a motor (68) with at least two motor windings (72a, 72b, 72c), and a memory device (90) for storing data characterizing the drive unit (14) and/or the motor (68), wherein the drive unit (14) comprises a first transmitting and receiving device (82a), which is connected to the memory device (90), and a second transmitting and receiving device (82b), which is connected to at least two connection contacts (70a, 70c) of the motor (68), and wherein the first and second transmitting and receiving devices (82a, 82b) are configured and interact in such a way that data are transferable between them in a contactless manner, **characterized in that** one (72c) of the at least two motor windings (72a, 72b, 72c) forms the second transmitting and receiving device (82b).

2. Surgical drive unit according to claim 1, **characterized in that** data stored in the memory device (90) are transferable in a contactless manner between the first and second transmitting and receiving devices (82a, 82b).

3. Surgical drive unit according to any one of the preceding claims, **characterized in that** the first and second transmitting and receiving devices (82a, 82b) are coupled in a contactless manner to each other.

4. Surgical drive unit according to any one of the preceding claims, **characterized by** a motor identification device (80) for identifying the kind or type of surgical drive unit (14) and/or of motor (68).

5. Surgical drive unit according to claim 4, **characterized in that** the motor identification device (80) comprises the first transmitting and receiving device (82a).

6. Surgical drive unit according to claim 4 or 5, **characterized in that** the motor identification device (80) is configured as an RFID (radiofrequency identification) component (86).

7. Surgical drive unit according to any one of the preceding claims, **characterized in that** electromagnetic high-frequency fields of short range are generatable with the first and/or second transmitting and receiving devices (82a, 82b).

8. Surgical drive unit according to any one of the preceding claims, **characterized in that** the drive unit (14) comprises a motor disabling device (98) for disabling operation of the motor (18).

9. Surgical instrument (60) comprising a drive unit (14) and a surgical tool (64) connected or connectable to the drive unit (14) and drivable by the drive unit (14), the drive unit (14) comprising a motor (68) with at least two motor windings (72a, 72b, 72c), and a memory device (90) for storing data characterizing the drive unit (14) and/or the motor (68), wherein the drive unit (14) comprises a first transmitting and receiving device (82a), which is connected to the memory device (90), and a second transmitting and receiving device (82b), which is connected to at least two connection contacts (70a, 70c) of the motor (68), and wherein the first and second transmitting and receiving devices (82a, 82b) are configured and interact in such a way that data are transferable between them in a contactless manner, **characterized in that** one (72c) of the at least two motor windings (72a, 72b, 72c) forms the second transmitting and receiving device (82b).

10. Surgical instrument according to claim 9, **characterized in that** the drive unit (14) is a surgical drive unit (14) according to one of claims 2 to 8.

11. Surgical drive system (10) comprising at least one control device (12) and at least two surgical drive units (14) connectable to and controllable by said control device (12), or surgical instruments (60) comprising surgical drive units (14), wherein at least one of the at least two surgical drive units (14) comprises a motor (68) with at least two motor windings (72a, 72b, 72c), and a memory device (90) for storing data characterizing the drive unit (14) and/or the motor (68), wherein the drive unit (14) comprises a first transmitting and receiving device (82a), which is connected to the memory device (90), and a second transmitting and receiving device (82b), which is connected to at least two connection contacts (70a, 70c) of the motor, and wherein the first and second transmitting and receiving devices (82a, 82b) are configured and interact in such a way that data are transferable between them in a contactless manner, **characterized in that** one (72c) of the at least two motor windings (72a, 72b, 72c) forms the second transmitting and receiving device (82b).

12. Surgical drive system according to claim 11, **characterized in that** at least one of the at least two surgical drive units (14) is a surgical drive unit (14) according to one of claims 2 to 8.

13. Surgical drive system according to claim 11 or 12, **characterized in that** the control device (12) is so configured that it is possible, in a first inquiry mode, to detect whether a drive unit (14) is connected to the control device (12).

14. Surgical drive system according to any one of claims 11 to 13, **characterized in that** the control device (12) is so configured that when a drive unit (14) is connected to the control device (12), it is possible, in a second inquiry mode, using the first and second transmitting devices (82a, 82b), to read and transfer to the control device (12) the data stored in the memory device (90).

15. Surgical drive system according to any one of claims 11 to 14, **characterized in that** the control device (12) is so configured that when a drive unit (14) is connected to the control device (12), it is possible, in a third inquiry mode, by conducting a high-frequency current across the connection contacts (70a, 70b) connected to the motor disabling device (98), to detect whether the switch element (100) is open or closed.

## Revendications

1. Unité d'entraînement chirurgicale (14) d'un instrument chirurgical (60), qui comprend un moteur (68) avec au moins deux enroulements de moteur (72a, 72b, 72c) et un dispositif de mémoire (90) pour mémoriser des données caractérisant l'unité d'entraînement (14) et/ou le moteur (68), l'unité d'entraînement (14) comportant un premier dispositif d'émission et de réception (82a), qui est relié au dispositif de mémoire (90), et un deuxième dispositif d'émission et de réception (82b), qui est relié à au moins deux contacts de raccordement (70a, 70c) du moteur (68), et le premier et le deuxième dispositifs d'émission et de réception (82a, 82b) étant configurés et interagissant mutuellement de manière telle, que des données puissent être transmises entre eux, sans contact, **caractérisée en ce que** l'un (72c) desdits au moins deux enroulements de moteur (72a, 72b, 72c) forme le deuxième dispositif d'émission et de réception (82b).

2. Unité d'entraînement chirurgicale selon la revendication 1, **caractérisée en ce que** des données mémorisées dans le dispositif de mémoire (90) peuvent être transmises, sans contact, entre le premier et le deuxième dispositif d'émission et de réception (82a, 82b) .

3. Unité d'entraînement chirurgicale selon l'une des revendications précédentes, **caractérisée en ce que** le premier et le deuxième dispositif d'émission et de réception (82a, 82b) sont couplés mutuellement, sans contact.

4. Unité d'entraînement chirurgicale selon l'une des revendications précédentes, **caractérisée par** un dispositif d'identification de moteur (80) pour identifier le genre et le type de l'unité d'entraînement chirurgicale (14) et/ou du moteur (68).

5. Unité d'entraînement chirurgicale selon la revendication 4, **caractérisée en ce que** le dispositif d'identification de moteur (80) comprend le premier dispositif d'émission et de réception (82a).

6. Unité d'entraînement chirurgicale selon la revendication 4 ou la revendication 5, **caractérisée en ce que** le dispositif d'identification de moteur (80) est réalisé sous la forme d'un composant RFID (Radio Frequency Identification) (86).

7. Unité d'entraînement chirurgicale selon l'une des revendications précédentes, **caractérisée en ce qu'**avec le premier et/ou le deuxième dispositif d'émission et de réception (82a, 82b) il est possible de produire des champs électromagnétiques haute-fréquence de faible portée.

8. Unité d'entraînement chirurgicale selon l'une des revendications précédentes, **caractérisée en ce que** l'unité d'entraînement (14) comporte un dispositif de blocage moteur (98) pour bloquer un fonctionnement du moteur (18).

9. Instrument chirurgical (60) comprenant une unité d'entraînement (14) et un outil chirurgical (64) qui est relié ou peut être relié à l'unité d'entraînement (14) et peut être entraîné par l'unité d'entraînement (14), l'unité d'entraînement (14) comprenant un moteur (68) avec au moins deux enroulements de moteur (72a, 72b, 72c) et un dispositif de mémoire (90) pour mémoriser des données caractérisant l'unité d'entraînement (14) et/ou le moteur (68), l'unité d'entraînement (14) comportant également un premier dispositif d'émission et de réception (82a), qui est relié au dispositif de mémoire (90), et un deuxième dispositif d'émission et de réception (82b), qui est relié à au moins deux contacts de raccordement (70a, 70c) du moteur (68), et le premier et le deuxième dispositifs d'émission et de réception (82a, 82b) étant configurés et interagissant mutuellement de manière telle, que des données puissent être transmises entre eux sans contact, **caractérisé en ce que** l'un (72c) desdits au moins deux enroulements de moteur (72a, 72b, 72c) forme le deuxième dispositif d'émission et de réception (82b).

10. Instrument chirurgical selon la revendication 9, **caractérisé en ce que** l'unité d'entraînement (14) est une unité d'entraînement chirurgicale (14) selon l'une des revendications 2 à 8.

11. Système d'entraînement chirurgical (10) comprenant au moins un dispositif de commande et/ou de régulation (12) auquel peuvent être reliés et par lequel peuvent être commandés au moins deux unités d'entraînement chirurgicales (14) ou instruments chirurgicaux (60) comprenant des unités d'entraînement chirurgicales (14), au moins l'une desdites au moins deux unités d'entraînement (14) comportant un moteur (68) avec au moins deux enroulements de moteur (72a, 72b, 72c) et un dispositif de mémoire (90) pour mémoriser des données caractérisant l'unité d'entraînement (14) et/ou le moteur (68), l'unité d'entraînement (14) comportant un premier dispositif d'émission et de réception (82a), qui est relié au dispositif de mémoire (90), et un deuxième dispositif d'émission et de réception (82b), qui est relié à au moins deux contacts de raccordement (70a, 70c) du moteur, et le premier et le deuxième dispositifs d'émission et de réception (82a, 82b) étant configurés et interagissant mutuellement de manière telle, que des données puissent être transmises entre eux sans contact, **caractérisé en ce que** l'un (72c) desdits au moins deux enroulements de moteur (72a, 72b, 72c) forme le deuxième dispositif d'émission et de réception (82b).

12. Système d'entraînement chirurgical selon la revendication 11, **caractérisé en ce que** l'une au moins desdites au moins deux unités d'entraînement chirurgicales (14) est une unité d'entraînement chirurgicale (14) selon l'une des revendications 2 à 8.

13. Système d'entraînement chirurgical selon la revendication 11 ou la revendication 12, **caractérisé en ce que** le dispositif de commande et/ou de régulation (12) est conçu pour, dans un premier mode de consultation, pouvoir détecter si une unité d'entraînement (14) est reliée au dispositif de commande et/ou de régulation (12).

14. Système d'entraînement chirurgical selon l'une des revendications 11 à 13, **caractérisé en ce que** le dispositif de commande et/ou de régulation (12) est conçu pour permettre, dans un deuxième mode de consultation, lorsqu'une unité d'entraînement (14) est reliée au dispositif de commande et/ou de régulation (12), la lecture des données mémorisées dans le dispositif de mémoire (90) et leur transmission au dispositif de commande et/ou de régulation (12) en utilisant le premier et le deuxième dispositif d'émission et de réception (82a, 82b).

15. Système d'entraînement chirurgical selon l'une des revendications 11 à 14, **caractérisé en ce que** le dispositif de commande et/ou de régulation (12) est conçu pour, dans un troisième mode de consultation, lorsqu'une unité d'entraînement (14) est reliée au dispositif de commande et/ou de régulation (12), pouvoir détecter si l'élément de commutation (100) est ouvert ou fermé, en faisant passer un courant haute fréquence par les contacts (70a, 70b) reliés au dispositif de blocage de moteur (98).
